# EUROPEAN PATENT APPLICATION

(11) **EP 0 651 996 A1**
(43) Date of publication of application: **10.05.1995**
(21) Application number: 94117403.9
(22) Date of filing: 04.11.1994
(51) Int. Cl.: A61K 9/16, C12N 11/04, B01J 13/04

(54) **Equipment and process for the preparation of polymeric microcapsules containing cells producing hormones**

(30) Priority: 08.11.1993 IT MI932371
(71) Applicant: Brotzu, Giovanni, I-09127 Cagliari (IT)
(72) Inventor: Brotzu, Giovanni, I-09127 Cagliari (IT)
(74) Representative: Gervasi, Gemma, Dr.

(57) **Abstract**

Equipment and process for the preparation of immunoinsulating polymeric microcapsules containing cells producing hormones. Said equipment comprises two coaxial tubes vertically disposed of which the inner tube (1) is provided for the oxygen feeding and the outer tube (2) is provided for the feeding of a cells suspension in a polymeric material. The product coming out from the two tubes system is picked up in the underlying basin (3) containing a solution suitable to induce the gelification of the polymeric material. Microcapsules of diameter between 10 and 500 µm according to the encapsulated cellular structures dimensions and having a ratio by volume with polymeric material and cells between 5:1 and 10:1 are obtained.

## Description

### PRIOR ART

Microcapsules formed by polymeric materials containing cells producing hormones are known.

Microcapsules of such a kind are produced for example by Darman Biotech (Boston) and commercialized with the name Encapcell.

Their features allow the protection of the cells contained in them against possible rejection phenomena, a correct metabolic activity and the release of the hormones secreted by the cells themselves.

However, the introduction of said microcapsules in the human body implies some problems among which the following ones can be pointed out:
1) The microcapsules introduced in the human body are wrapped up by a connective reactive tissue preventing whether the metabolic supply to the cells or the emission of the hormones produced by them;
2) The microcapsules, injected in a cavity such as peritoneum, tend to deposit in the cavity bottom concentrating in a restricted zone and determining a lively reaction of connective kind wrapping them up.

These problems could be solved making suitable vascular protheses enclosing in their walls, of semipermeable kind, the microcapsules. These protheses, planted in the recipient in order to form some arterial-venous fistulas, would allow the microcapsules exposition to the hematic flux carrying out in this way a correct metabolism of the cells contained in the microcapsules and the release in circle of hormones secreted by the cells themselves.

However, this solution turns out to be impossible with the use of microcapsules obtainable with the prior art as the protheses would result too voluminous in order to be applied.

In fact the volume of the polymeric material needed to encapsulate the cells turns out to be from 60 to 100 times the volume of the cells themselves and this makes impossible the realization of vascular protheses of acceptable dimensions. Possible protheses made with the microcapsules of the prior art would have a minumum length of 600 mm and a minimum diameter of 12 mm.

### SUMMARY

Now it has been surprisingly found that it is possible to prepare immunoinsulating microcapsules containing cells producing hormones using a polymeric material volume between 5 and 10 times the volume of the cells themselves.

This result has been possible thanks to the equipment and process of the present invention.

The equipment comprises a system of two coaxial tubes vertically disposed and an underlying basin.

The inner tube is connected to a system for the oxygen feeding and shows the lower end retracted with respect to the same end of the outer tube while the outer tube, on the upper part bound to the inner tube circumference, is connected with a system for the feeding of a cells suspension in a polymeric material.

The underlying basin is arranged to contain a solution suitable to induce the gelification of the droplets of the polymeric suspension coming out from the two tubes system.

The process of the present invention consists in feeding to said tubes system oxygen and a cells suspension in a polymeric material and in picking up in said basin the product coming out from the tubes system and it is characterized by:
a) A suspension of cells in a polymeric material with a viscosity of at least 300 cps is prepared;
b) Said oxygen is fed through the inner tube with a velocity between 75 and 95 m/s;
c) Said suspension is fed through the interspace limited by the two tubes with a velocity which in the interspace itself has a value between 3 and 5 mm/s;
d) The product coming out from the tubes system droplets-shaped is picked up in said basin containing a solution suitable to induce the polymeric material gelification obtaining in this way the stable microcapsules containing the cells.

The microcapsules so obtained, thanks to the high cells content, are suitable to be used for the realization of vascular protheses having dimensions suitable to be planted in the human body.

### BRIEF DESCRIPTION OF THE DRAWING

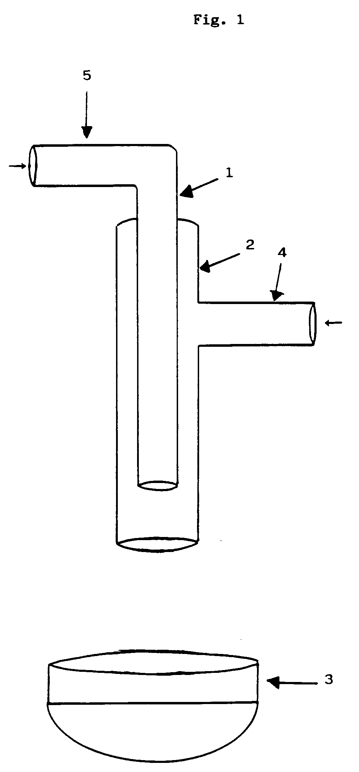

Figure 1 represents the equipment according to the present invention, essentially consisting of two coaxial tubes and of an underlying basin.

### DETAILED DESCRIPTION OF THE INVENTION

The features and profits of the equipment and process for the preparation of the immunoinsulating microcapsules containing cells producing hormones according to the present invention, will be mostly pointed out by the following detailed description.

Referring to the numerical symbols of the Fig.1 said equipment essentially comprises two coaxial tubes, respectively 1 and 2 and a basin 3.

The outer tube 2 is bound, at the same level of its upper end, to the inner tube 1 circumference and moreover it is provided with a side tube 4 which is connected to a system for the feeding of a cells suspension in a polymeric material. The inner tube 1, in turn, is connected by the side extension 5 with a system for the oxygen feeding.

The lower end of the tube 1 is retracted of a length between 0.8 and 1.5 times its inner diameter with respect to the lower end of the tube 2. The distance between the tube 4 axis and the lower end of the tube 2 is between 4 and 8 times the diameter of the tube 2 itself.

The area of the interspace cross section limited by the two tubes 1 and 2 has a value between 5 and 10 times the area of the inner tube 1 cross section.

The tubes system is made of special metals such as stainless alloy or titanium or of an engineering kind polymeric material.

The basin 3 contains a solution suitable to induce the gelification of the polymeric material used for the cells suspension and the distance between said solution surface and the lower end of the tube 2 has a value between 100 and 150 times the diameter of the tube 2 itself.

The cellular suspension is prepared by accurate dispersion of the cells in a polymeric material having a viscosity between 250 and 360 cps.

The cells may be in the shape of single cells or in the shape of cellular complexes. Therefore, for the microcapsules of the present invention, Langherans islands containing beta cells, thyroidal or suprarenal cellular complexes, hepatocytes, erythrocytes and other cells may be used.

Favourite polymeric material is sodium alginate however other polysaccharide or synthetic materials may be also used.

The ratio by volume between said polymeric material and said cellular complexes or cells is between 5:1 and 10:1.

The solution suitable to induce the polymeric material gelification (Na alginate) is in preference an acqueous BaCl₂ solution with a concentration between 2.0 and 2.4 millimoles per liter.

Instead of BaCl₂ another salt of a polyvalent metal may be also used.

For the preparation of the microcapsules according to the present invention the cellular suspension described above is fed through the tube 4 at such a flow rate in the interspace limited by the tube 1 and 2 that said suspension flows with a velocity between 3 and 5 mm/s. At the same time oxygen is fed through the tube 5 at such a rate as to flow through the tube 1 with a velocity between 75 and 95 m/s.

The droplets coming out from the tube 2 are picked up in the solution contained in the basin 3 in which the gelification of the polymeric material occurs.

The immunoinsulating microcapsules containing cells secreting hormones according to the present invention which have the shape of microspheres having a diameter between 10 and 500 µm are thus obtained.

The microcapsules are divided from the BaCl₂ solution by centrifugation. In the microcapsules of the present invention every cell is totally coated with the polymeric material. With the microcapsules of the present invention it is possible to make vascular protheses having length 120 mm and diameter 12 mm.

The following examples are reported for explanatory but not limitative purpose.

### EXAMPLE 1

A preparation of microcapsules containing Langherans islands in sodium alginate has been carried out.

An equipment as shown in Fig.1 in which the tube 1 had the inner diameter equal to 0.8 mm and the outer diameter equal to 1.2 mm and the tube 2 had the inner diameter equal to 2.5 mm, has been used.

Oxygen at a rate of 2.5 Nl per minute corresponding to a velocity equal to 83 m/s in the tube 1 was fed through the tube 5. A suspension of Langherans islands in sodium alginate with a viscosity equal to 350 cps at a rate of 1 ml per minute corresponding to a velocity equal to 4.4 mm/s in the interspace limited by the tubes 1 and 2 was fed through the tube 4.

The islands content with respect to the polymeric material was equal to 1:6 by volume.

The droplets coming out from the tube 2 were picked up in the underlying basin 3 in an acqueous BaCl₂ 2.2 mM solution whose surface was 30 cm to the lower end of the tube 2.

The obtained microcapsules have been separated from the BaCl₂ solution by centrifugation.

In the obtained product every island turned out to be uniformly coated with alginate and had a diameter between 50 and 500 µm.

### EXAMPLE 2 (comparison)

The example 1 has been repeated with the only difference that the Langherans islands suspension has been carried out in sodium alginate with a viscosity equal to 150 cps.

A product consisting of microcapsules of heterogeneous shapes and containing a lot of non-encapsulated islands has been obtained.

### EXAMPLE 3 (comparison)

The example 1 has been repeated with the only difference that the oxygen has been fed at a rate equal to 0.8 Nl per minute corresponding to a velocity equal to 26.6 m/s along the tube 1. A product consisting of microcapsules
of huge dimensions with an average diameter equal or greater than 700 µm has been obtained.

### EXAMPLE 4

A preparation of microcapsules containing erythrocytes has been done. An equipment as in the Fig. 1 in which the tube 1 had an inner diameter equal to 0.8 mm and an outer diameter equal to 1.2 mm and the tube 2 had an inner diameter equal to 2.5 mm has been used. Oxygen at a rate equal to 2.5 Nl/min corresponding to a velocity equal to 83 m/s in the tube 1 was fed through the tube 5.

An erythrocytes suspension in sodium alginate with a viscosity equal to 350 cps at a rate equal to 1 ml/min corresponding to a velocity equal to 4.4 mm/s in the interspace limited by the tubes 1 and 2 was fed through the tube 4.

The erythrocytes content with respect to the polymeric material was equal to 1:10.

The droplets coming out from the tube 2 were picked up in the underlying basin in an acqueous BaCl₂ 2.2 mM solution, whose surface was 30 cm to the lower end of the tube 2.

The obtained microcapsules have been divided from BaCl₂ by centrifugation. In the obtained product every erythrocyte was uniformly coated with alginate and the microcapsules had a diameter between 15 µm (when containing only one erithrocyte) and 250 µm (when containing thousands erythrocytes).

The microcapsules have been exposed to serum containing hemolysins belonging to the IgG (MW 146.000) class and at the level of the erythrocytes contained inside the microcapsules no hemolysis sign has been pointed out, thus showing the immunoinsulation carried out by abovementioned microcapsules.

## Claims

1. Equipment for the preparation of immunoinsulating polymeric microcapsules containing cells producing hormones comprising two coaxial tubes (1) and (2) vertically disposed and an underlying basin (3), the outer tube (2) being bound at the level of its upper end to the inner tube (1), characterized by the fact that the inner tube is connected to a system for the oxygen feeding and has the lower end retracted with respect to the similar end of the outer tube and that the outer tube is connected to a system for the feeding of a cells suspension in a polymeric material.

2. Equipment as claimed in claim 1, characterized by the fact that the area of the interspace cross section limited by said tubes has a value between 5 and 10 times the area of the inner tube cross section.

3. Equipment as claimed in claim 1, characterized by the fact that said retraction of the inner tube end with respect to the similar outer tube end has a length between 0.8 and 1.5 times the inner diameter of said inner tube.

4. Process for the preparation of immunoinsulating polymeric microcapsules containing cells producing hormones by the equipment as claimed in claims from 1 to 3 to which oxygen and a cells suspension in a polymeric material are fed, characterized by the fact that:
a) a cells suspension in a polymeric material with a viscosity at least 250 cps is prepared;
b) oxygen through said inner tube at such a rate as to obtain a flux velocity between 75 and 95 m/s is fed;
c) said suspension through the interspace limited by said two coaxial tubes at such a rate as to obtain a flux velocity between 3 and 5 mm/s is fed;
d) the product coming out from the system of said tubes in shape of droplets is picked up in said basin containing a solution suitable to induce the polymeric material gelification.

5. Process as claimed in claim 4, characterized by the fact that the viscosity of said polymeric material is between 250 and 360 cps.

6. Process as claimed in claim 4, characterized by the fact that the polymeric material is sodium alginate.

7. Process as claimed in claim 4, characterized by the fact that said cells have the shape of single cells or of cellular complexes and are selected in the group including Langherans islands, thyroidal and suprarenal cellular complexes, hepatocytes and erythrocytes.

8. Process as claimed in claim 4, characterized by the fact that the ratio by volume between said polymeric material and said cells is between 5:1 and 10:1.

9. Process as claimed in claim 4, characterized by the fact that said solution suitable to induce the polymeric material gelification is an acqueous BaCl₂ solution with a concentration between 2.0 and 2.4 millimoles per liter.

10. Polymeric microcapsules containing cells producing hormones characterized by the fact to be formed by microspheres having a diameter between 10 and 500 µm with a ratio by volume with the polymeric material and cells between 5:1 and 10:1.
